# EUROPEAN PATENT SPECIFICATION

(11) **EP 2 826 561 B1**
(45) Date of publication and mention of the grant of the patent: **16.06.2021**
(21) Application number: 13760473.2
(22) Date of filing: 13.03.2013
(51) Int. Cl.: B01J 23/85, B01J 23/28, B01J 23/30, B01J 23/88, B01J 37/02, B01J 37/08, C07C 67/03, C07C 67/08, C07C 69/24, C07C 69/58, C07C 69/587, C07B 61/00, B01J 23/34, B01J 23/882, B01J 23/888, C11C 1/10

(54) **METHOD FOR MANUFACTURING A FATTY ACID ALKYL ESTER USING A SOLID ACID CATALYST**
VERFAHREN ZUR HERSTELLUNG EINES FETTSÄUREALKYLESTERS MIT EINEM FESTEN SÄUREKATALYSATOR
PROCÉDÉ DE FABRICATION D'UN ESTER ALKYLIQUE D'ACIDE GRAS UTILISANT UN CATALYSEUR ACIDE SOLIDE

(30) Priority: 13.03.2012 JP 2012055461; 13.03.2012 JP 2012055462
(43) Date of publication of application: 21.01.2015
(73) Proprietor: Benefuel Inc., Irving TX 75039 (US)
(72) Inventor: OH Shosei, Matsuyama-shi Ehime 791-8022 (JP)
(74) Representative: Elsy, David
(86) International application number: PCT/JP2013/056908
(87) International publication number: WO 2013/137286

(56) References cited:
- WO-A1-2011/018802
- JP-A- S57 118 533
- JP-A- 2006 218 358
- JP-A- 2012 024 654
- US-A- 2 437 532
- US-A- 2 748 062
- US-A- 3 014 066
- US-A- 3 329 826
- US-A- 3 867 412
- US-A- 4 408 067
- US-A- 4 464 539

## Description

### Technical Field

The present invention employs a solid acid catalyst having high activity, and activity stability, for reactions catalyzed by Lewis acids or Broensted in a method of manufacturing fatty acid alkyl esters using the same.

### Background Art

Fatty acid alkyl esters are used as pharmaceutical products, starting materials for resins and chemicals, and as alternative fuels for petroleum light oils and the like.

Fatty acid alkyl esters are normally manufactured by an esterification reaction of a fatty acid and a C₁ to C₁₀ lower alcohol, or by an ester exchange reaction of a fatty triglyceride and a C₁ to C₁₀ lower alcohol. Industrially, these are manufactured by way of a method in which fatty triglycerides, which are the principal components of vegetable oils and animal oils, serve as the starting material and a reaction is performed by way of ester exchange with the vegetable oils or animal oils in an alcohol solvent, with an alkali as a catalyst, so as to manufacture a fatty acid monoester.

However, because plant oils and animal oils generally contain free fatty acids, in methods of manufacturing fatty acid monoesters performed by way of adding an alkali catalyst as described above, the free fatty acids and the alkali catalyst react before the ester exchange reaction takes place, producing soaps and water. The water greatly reduces the alkali catalyst action, and the soap that is produced acts as a surface active agent, which makes separation of the product and the catalyst difficult. Meanwhile, acid catalysts such as sulfuric acid are capable of catalyzing free fatty acid esterification reactions and triglyceride ester exchange reactions at the same time, but because the rate of the ester exchange reaction is markedly slower than the esterification reaction, there are few examples of use in industry.

Recently, research is actively underway into the manufacture of fatty acid alkyl esters using solid acid or solid base catalysts. Among these, because solid acid catalysts are capable of catalyzing free fatty acid esterification reactions and triglyceride ester exchange reactions at the same time, there are no limits on the content of free fatty acids in the starting material oils and fats, and separation of the catalyst after the reaction is easy.

Heretofore, solid acid catalysts such as zeolites, ion exchange resins and heteropolyacids have been studied, but the acidity of zeolite catalysts is low, and because the movement of substances within the fine pores is limited, the catalytic activity is low. Ion exchange resins such as sulfonic acid resins require reaction temperatures of 170°C or more in order to increase their activity, but the resin is unable to withstand such temperatures. Meanwhile heteropolyacid catalysts present a problem in so much as they are readily soluble in water so that the active components are leached in a short period of time, such that activity is lost.

Meanwhile, metallic oxide catalysts such as TiO₂, ZrO₂ and TiO₂-ZrO₂ impregnated with acid (JP-09-103681-A, JP-11-244701-A, JP-11-057478-A) and amorphous carbon into which a sulfonic acid group has been introduced (JP-2009-114272-A) both demonstrate activity for esterification reactions and ester exchange reactions, but as with heteropolyacid catalysts, there is a disadvantage in that the sulfate radical is readily leached. Furthermore, in the case of amorphous carbon into which a sulfonic acid group has been introduced, it is difficult to work the catalyst to the form and strength required for a solid bed circulation reactor, making this unsuitable for industrial production apparatus.

In terms of other solid acid catalysts, a solid acid catalyst characterized by containing α-alumina and tungstic acid, having a specific surface area of 3 to 50 m²/g, and an argon absorption heat of no greater than -14.5 kJ/mol (JP-2007-175649-A), a solid acid catalyst resulting from supporting molybdenum oxide on a zirconia carrier and firing at 673 K to 1473 K (JP-2009-149900-A), and a solid acid catalyst having: a metal oxide layer wherein metal oxides of niopium and/or tantalum and molybdenum and/or tungsten form layered structures; and protons, which are present between the metal oxide layers (JP-2007-229627-A) have been disclosed. While these solid acid catalysts demonstrate a certain activity for both fats and alcohols, in particular in the case of oil and fat starting materials having high free fatty acid contents, there is a problem in so much as the free fatty acids work as reducing agents, which reduce and elute the metal components such as molybdenum and tungsten, which are the active components, so that catalytic activity is lost in a short time US-A-3 329 826 also discloses solid acid catalysts for making fatty acid alkyl esters.

### Prior Art Documents

### Patent Documents

Patent Document 1: JP-09-103681-A
Patent Document 2: JP-11-244701-A
Patent Document 3: JP-11-057478-A
Patent Document 4: JP-2009-114272-A
Patent Document 5: JP-2007-175649-A
Patent Document 6: JP-2009-149900-A
Patent Document 7: JP-2007-229627-A

### Summary of the Invention

### Problems to Be Solved by the Invention

An object of the present invention is to provide a method for manufacturing a fatty acid alkyl using using a solid acid catalyst having high activity for acid catalyzed reactions such as esterification reactions, ester exchange reactions, alkylation reactions and isomerization reactions, with which reaction temperatures are low, secondary reactions are minimized, and there is no leaching of reaction components during the reaction.

### Means for Solving the Problems

The method of manufacturing a fatty acid alkyl ester in accordance with the invention is a method of manufacturing a fatty acid alkyl ester by reacting a fatty acid and/or a triglyceride and an alcohol in the presence of the solid acid catalyst recited in claim 1 including: a first reaction step of producing a fatty acid alkyl ester reaction solution A by contacting the fatty acid and/or the triglyceride and the alcohol with the solid acid catalyst at a temperature of 100 to 250°C and a pressure of 0.1 to 6.0 MPa; a first separation step, following on said first reaction step, of removing said alcohol, water and glycerol from said fatty acid alkyl ester reaction solution A, to produce a crude fatty acid alkyl ester A having a water concentration of no greater than 0.1%; a second reaction step of contacting said crude fatty acid alkyl ester A and said alcohol with the solid acid catalyst at a temperature of 60 to 210°C at a pressure of 0.1 to 6.0 MPa (WE USE 1-4 MPa); and a second separation step of further removing said alcohol, water and glycerol from a fatty acid alkyl ester B produced in the second reaction step, to produce a fatty acid alkyl ester B having a free glycerol concentration of no greater than 0.02%.

The method of manufacturing a fatty acid alkyl may include an ester distillation step of distilling the crude fatty acid alkyl ester B produced in the second separation step under reduced pressure so as to cut the fractions having boiling points less than or equal to 100°C and greater than or equal to 360°C, to produce a refined fatty acid alkyl ester.

In the method of manufacturing a fatty acid alkyl ester the molar ratio of the alcohol with respect to the fatty acid and/or triglyceride, calculated as an alcohol to fatty acid molar ratio, may be from 1.2 to 40, and in said second step, the molar ratio of the alcohol with respect to the crude fatty acid alkyl ester, calculated as an alcohol to fatty acid molar ratio is from 1.1 to 30.

The method of manufacturing a fatty acid alkyl ester may be performed such that in said first separation step and said second separation step, the glycerol is separated after heating the fatty acid alkyl ester reaction solution A or the fatty acid alkyl ester reaction solution B to a temperature higher than the boiling point of either said alcohol or water at ordinary pressure or under reduced pressure, and evaporating said alcohol and the water.

The method of manufacturing a fatty acid alkyl ester may be performed such that in said first reaction step, the reaction is performed with the addition of 0 to 3% water, with respect to the fatty acid and/or triglyceride that is the starting material.

### Effects of the Invention

With the catalyst used in the present invention, fatty acid alkyl esters can be efficiently manufactured, without requiring rigorous operating conditions, simply by contacting the solid catalyst with a mixture of an animal or vegetable oil or fat principally comprising fatty acids and/or triglycerides and a lower alcohol. As compared to conventional homogeneous phase reaction processes using an alkali or acid catalyst, this solid acid catalyst produces high purity fatty acid alkyl esters and glycerol without limitations on the oil and fat starting materials, without the need for a step of removing the catalyst from the reaction product, with little waste generated, and at high yields. Furthermore, as compared to conventionally proposed solid catalysts, the catalytic activity is high, there are few secondary reactions, there is little loss in activity due to effusion of catalyst components, and the catalyst life is long.

Here, the following advantages result from the present invention. As a result of adding a co-catalyst selected from the group consisting of manganese (Mn), iron (Fe), cobalt (Co), nickel (Ni), copper (Cu), zinc (Zn), gallium (Ga) and tin (Sn) to the principal catalyst component that is selected from group Vlb of the periodic table, the catalyst activity is greatly increased, and reactions can be performed at lower temperatures. Consequently, secondary reactions can be minimized. Moreover, the boron (B) or silicon (Si) nonmetallic oxide serves to prevent the catalyst active components from being dissolved into the reaction fluid and flowing out, allowing the stability of the catalyst to be improved.

Note that, with the manufacturing method of the present invention, a high-quality fatty acid alkyl ester with little free fatty acid residue can be manufactured at high yields by including: a first separation step, following on the first reaction step, of removing the alcohol, water and glycerol from the fatty acid alkyl ester reaction solution A, to produce a crude fatty acid alkyl ester A having a water concentration of no greater than 0.1 %; and a second reaction step of contacting the crude fatty acid alkyl ester A and the alcohol with the solid acid catalyst at a temperature of 60 to 210°C at a pressure of 0.1 to 6.0 MPa.

### Brief Description of the Drawings

**[****FIG. 1****]** This is a block diagram showing the method of manufacturing fatty acid alkyl esters of the present invention.
**[****FIG. 2****]** This is a configuration diagram of a device for manufacturing fatty acid alkyl esters used in a working example of the present invention.

### Modes of Embodiment of the Invention

Hereafter, the present invention will be described in detail.

In terms of examples of starting materials used for the method of manufacturing fatty acid alkyl esters using the same, various animal and vegetable oils and fats principally comprising triglycerides, and fatty acids resulting from hydrolysis of animal and vegetable oils and fats may be mentioned as being representative.

Vegetable oils include soybean oil, rapeseed oil, sunflower oil, cottonseed oil, coconut oil, sesame oil, olive oil, corn oil, peanut oil, castor oil, rice oil, palm oil, Jatropha oil, algal oil, and the like. Animal oils include beef tallow, lard, horse fat, fish oil, whale oil, and the like. These oils and fats may be each alone, or in mixtures of two or more thereof. Note that these oils and fats may be used waste oils. Examples of used waste oil include waste oils and fats discarded from oil processing plants, food manufacturing plants, restaurants, general households, and the like; oil and fat residues in edible oil manufacturing processes such as oil cakes; waste oils and fats such as vegetable oils and fats used as lubricating oils in metal hot rolling; waste oils and fats that occur in processing oils and fats such as margarine and shortening; edible oils and fats in returned goods such as defective products and expired products, animal oils and fats that occur in edible oil fish-meat processing processes and the like.

The alcohol used for the solid acid catalyst, the method of manufacturing the same and the method of manufacturing fatty acid alkyl esters using the same according to the present invention preferably consists of a C₁₋₁₀ saturated aliphatic hydrocarbon group. As such alcohols, primary alcohols such as methanol, ethanol, n-propanol and n-butanol, secondary alcohols such as isopropanol and sec-butanol, and tertiary alcohols such as tert-butanol can be used. Among these, primary alcohols such as methanol and ethanol are particularly preferred. Note that there are no particular limits on the water content of these alcohols, but the lower the water content, the more preferable it is.

With the solid acid catalyst, the method for manufacturing the same, and the method of manufacturing fatty acid alkyl esters using the same according to the present invention, if a triglyceride and an alcohol are reacted, the fatty acid alkyl ester can be produced by the reaction represented by Chem. 1. Furthermore, if a fatty acid and alcohol are reacted, the fatty acid alkyl ester can be produced by the reaction represented by Chem. 2.

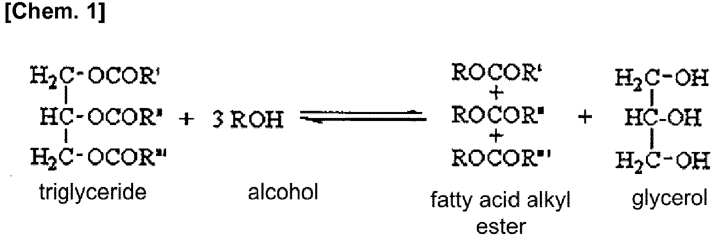

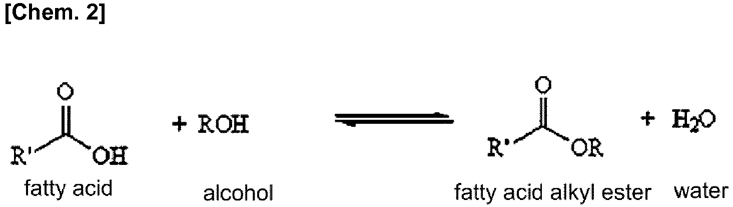

The solid acid catalyst of the present invention is produced by supporting, on the inorganic porous carrier (A): an oxide (B) of at least one metallic element selected from the Vlb group of the periodic table; an oxide or sulfate (C) of at least one metallic element selected from the group consisting of manganese (Mn), iron (Fe), cobalt (Co), nickel (Ni), copper (Cu), zinc (Zn), gallium (Ga) and tin (Sn); and an oxide (D) of at least one nonmetallic element selected from boron (B) and silicon (Si).

In the present invention, alumina or silica are used, but if only one is selected, alumina (Al₂O₃) is most preferable. If two or more are selected, silica-alumina (SiO₂-Al₂O₃) is more preferred, as the performance is higher than with only one of either silica or alumina.

Thus, the solid acid catalyst according to the present invention is such that, as active metal components, with respect to the total amount of catalyst, 2.5 to 25 mass% of the VIb group metallic element as calculated for the oxide; with respect to the total amount of catalyst, 1 to 10 mass% of the oxide or sulfate (C) of at least one metallic element selected from the group consisting of manganese (Mn), iron (Fe), cobalt (Co), nickel (Ni), copper (Cu), zinc (Zn), gallium (Ga) and tin (Sn) as calculated for the oxide; and with respect to the total amount of catalyst, 0.5 to 5 mass% of the at least one nonmetallic element selected from boron (B) and silicon (Si) as calculated for the oxide, are supported on the inorganic porous carrier.

In the present invention, the group Vlb metallic element is usually selected from chromium, tungsten and molybdenum. These metallic elements are supported on the carrier as metal oxides. There are no particular limits on the oxidation states but Cr₂O₃, CrO₂, CrO₃, MoO₂, MoO₃, WO₂, WO₃ and the like can be mentioned. These metal oxides may be used alone, or may be used in mixtures of two or more. There are no particular limitations on the methods of supporting or mixing, but ordinary impregnation methods and methods of mixing in the solid phase and the like can suitably be used.

There are no particular limitations on the starting materials for supporting the group VIA metal element on the carrier as an oxide, but for example, ammonium chromate, chromium nitrate, ammonium tungstate, ammonium metatungstate, ammonium molybdate, tungstic acid, and tungsten chloride can be used. These compounds may be used alone, or in combinations of two or more.

The oxide or sulfate (C) of the metallic element that is supported on the catalyst carrier together with the group Vlb metallic element is preferably selected from the group consisting of manganese (Mn), iron (Fe), cobalt (Co), nickel (Ni), copper (Cu), zinc (Zn), gallium (Ga) and tin (Sn), and the use of tin and zinc is preferred. These metallic elements are supported on the carrier as oxides or sulfates.

There are no particular limitations on the starting material for supporting the metal selected from manganese (Mn), iron (Fe), cobalt (Co), nickel (Ni), copper (Cu), zinc (Zn), gallium (Ga) and tin (Sn) on the carrier as an oxide or sulfate in this manner but, for example, the use of sulfuric acid salts, nitric acid salts, carbonic acid salts, acetic acid salts, phosphoric acid salts or the like is preferred. In particular, sulfuric acid salts are the most preferred for obtaining the metal sulfate. These compounds may be used alone, or in combinations of two or more.

Furthermore, as the oxide of the nonmetallic element that is supported on the catalyst carrier together with the group Vlb metallic element and the metal selected from the group consisting of manganese (Mn), iron (Fe), cobalt (Co), nickel (Ni), copper (Cu), zinc (Zn), gallium (Ga) and tin (Sn), one or two or more nonmetallic oxides selected from boron (B) and silicon (Si) can be mentioned. Specifically, boric acid, silicic acid, ethyl silicic acid and the like can be mentioned, but among these the use of boric acid and ethyl silicic acid is particularly preferred. Boron and silicon are normally supported on the carrier as oxides

According to the present invention the amount of these active components supported is, as calculated for the oxides, and based on the weight of the carrier, 2.5 to 25 mass% and preferably 5 to 15 mass% of the metal oxide (B), 1 to 10 mass% and preferably 2 to 5 mass% of the metal oxide or metal sulfate (C), and 0.5 to 5 mass%, and preferably 1 to 2.5 mass% of the non-metal oxide (D), and the total of B, C and D is no greater than 30%.

When the amount of the group Vlb metal component supported by the catalyst is less than 2.5 mass%, sufficient catalyst activity is not produced, while even if the amount supported exceeds 25 mass%, the distribution of the active metals will be poor and the aforementioned catalyst activity will be saturated, and thus this is detrimental to the economics of catalyst manufacture.

Meanwhile, when the amount of the oxide or sulfate (C) of the metal element supported is less than 1 mass%, a sufficient effect as a co-catalyst will not be produced, and thus it will not be possible to effectively convert oils and fats to esters. However, even if more than 10 mass% is supported, the esterification activity will be saturated, and the catalytic activity will in fact be lowered, which is economically disadvantageous.

Next, the oxide of the nonmetallic element (D) is useful in improving the dispersion characteristics of the oxide (B) of the metal element and the oxide or sulfate (C) of the metal element on the carrier and increasing the active sites, as well as in preventing reduction of the metal oxides and elution from the catalyst. If the amount of the oxide (D) of the nonmetallic element supported is less than 0.5 mass%, it is not possible to effectively produce the aforementioned effect. However, even if 5 mass% is exceeded, the aforementioned effect will be saturated, and thus this will not be economical.

Note that the total of B, C and D is no greater than 30%, and preferably no greater than 25%. If the total of B, C and D exceeds 30%, the distribution of the active metals will be poor, and the aforementioned catalyst activity will be saturated, which is detrimental to the economy of the catalyst manufacture.

The method of impregnating the inorganic porous carrier (A) with the nonmetallic oxide (D) simultaneously with impregnating with the metal oxide (B) and the metal oxide (C) is used and has a pronounced effect.

That is to say, the catalyst according to the present invention can be produced by dissolving the compounds of group Vlb and of the at least one metallic element selected from the group consisting of manganese (Mn), iron (Fe), cobalt (Co), nickel (Ni), copper (Cu), zinc (Zn), gallium (Ga) and tin (Sn) in water to produce an aqueous solution with which the carrier is impregnated, subsequently heating and drying, preferably at 120°C for 2 to 24 hours, and then firing at 400 to 750°C for 2 to 24 hours.

There are no particular limitations on the form of the solid acid catalyst used in the present invention produced in this manner, which is to say the specific surface area, the pore volume and the average pore size, but in order to efficiently convert fatty acids and/or animal or vegetable oils and fats to fatty acid alkyl esters, it is preferable that the specific surface area be no less than 100 m²/g, that the pore volume be in the range of 0.3 to 1.2 cc/g, and that the average pore size be in the range of 60 to 120 Å.

Note that there are no particular limitations on the acidity of the solid acid catalyst used in the present invention, but in order to increase the conversion efficiency from fatty acids and/or animal or vegetable oils and fats to fatty acid alkyl esters, and limit other secondary reactions, it is preferable that the Hammett function H0, which indicates acidity, be -1.5 to -11, and -4 to -10 is more preferred. This is because, in this acidity range, the triglyceride and alcohol ester exchange reaction (Chem. 1) and the fatty acid and alcohol esterification reaction (Chem. 2) can progress smoothly with few secondary reactions, and thus fatty acid alkyl esters are produced from fatty acids and/or triglycerides and alcohol at high efficiencies.

According to the present invention, there are no particular limitations on the shape of the catalyst, but powders, extruded molds, tablet molds and the like can normally be used. Shapes for extrusion molds generally include cylinders, three-leafed shapes, four-leafed shapes, rings and the like, and while this is not particularly limited in the present invention, cylinders, three-leafed shapes and four-leafed shapes are preferred. With cylinders, three-leafed shapes and four-leafed shapes, catalytic packing can be performed more densely than with catalysts of other shapes such as rings, and crushing damage is also limited as compared to spheres and granules. Furthermore, in terms of size, diameters of 0.25 to 0.12 cm (1/10 to 1/22 inches) and lengths of 8.1 to 9.1 cm (3.2 to 3.6 inches) are normally preferred.

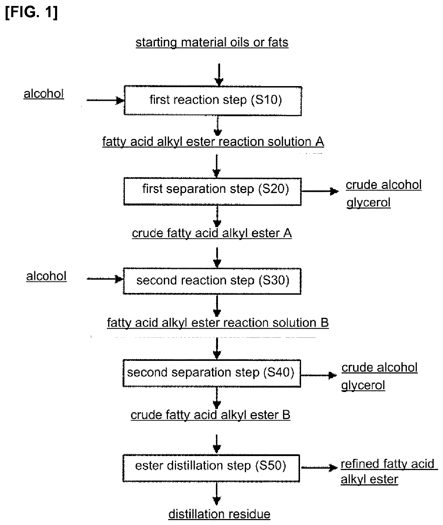

Next, the steps shown in FIG. 1 will be described in detail.

FIG. 1 is a block diagram showing the method of manufacturing fatty acid alkyl esters of the present invention and the method of manufacturing fatty acid alkyl esters with the manufacturing apparatus for the same.

As shown in the figure, the method of manufacturing fatty acid alkyl esters of the present invention comprises: a first reaction step (S10) of producing a fatty acid alkyl ester reaction solution A by contacting the fatty acid and/or the triglyceride and the alcohol with the solid acid catalyst at a temperature of 100 to 250°C and a pressure of 0.1 to 6.0 MPa; a first separation step (S20), following on the first reaction step, of separating and removing the alcohol, water and glycerol from the fatty acid alkyl ester reaction solution A, to produce a crude fatty acid alkyl ester A having a water concentration of no greater than 0.1%; a second reaction step (S30) of contacting the crude fatty acid alkyl ester A and the alcohol with the solid acid catalyst at a temperature of 60 to 210°C at a pressure of 0.1 to 6.0 MPa; and a second separation step (S40) of further removing the alcohol, water and glycerol from a crude fatty acid alkyl ester reaction solution B produced in the second reaction step, to produce a crude fatty acid alkyl ester B.

Next, the manufacturing method of the present invention will be described.

### (i) First reaction step (S10)

In the present invention, the fatty acid and/or the triglyceride and the alcohol are contacted with the solid acid catalyst at a temperature of 100 to 250°C, more preferably 120 to 230°C, and particularly preferably 140 to 210°C, and a pressure of 0.1 to 6.0 MPa, preferably 0.5 to 5 MPa, and particularly preferably 1.0 to 4.5 MPa. The esterification and ester exchange reactions will progress even at lower than 100°C, but the reaction rate will be slow and the production efficiency will be poor. Meanwhile, if the reaction temperature is greater than or equal to 250°C, secondary reactions other than the esterification and ester exchange reactions will be pronounced, and the fatty acid alkyl ester yield may in fact be lowered.

If the contact time of the fatty acid and/or triglyceride and the alcohol with the solid acid catalyst is expressed as weight space velocity (WHSV), this is 0.3 to 1.5 h⁻¹, and preferably 0.4 to 0.8 h⁻¹. Generally, the lower the WHSV, the longer the contact time will be and the greater the reaction rate, but even if the WHSV is less than or equal to 0.3 h⁻¹, it is only possible to increase the rate of secondary reactions, and this is undesirable from the point of view of productivity. If the WHSV exceeds 1.5 h⁻¹, the reaction rate will greatly decrease, which is undesirable from the point of view of productivity.

In the aforementioned esterification reaction, if 1 mole of alcohol is reacted for 1 mole of fatty acid, the corresponding mole of fatty acid alkyl ester is produced. Furthermore, in the aforementioned ester exchange reaction, if 3 moles of alcohol is reacted for 1 mole of triglyceride, the corresponding 1 mole of fatty acid alkyl ester is produced. However, both the esterification reaction and the ester exchange reaction are equilibrium reactions, and as the concentrations of the reaction products increase, reverse reactions become pronounced. In order to limit reverse reactions and cause forward reactions to advance to as great an extent as possible, it is effective to have an excess amount of the reactant alcohol. Accordingly, in the present invention, in the first step, the molar ratio of the alcohol with respect to the fatty acid and/or triglyceride, calculated as an alcohol to fatty acid molar ratio, is from 1.2 to 40, more preferably 1.5 to 30, and particularly preferably 3 to 15. Furthermore, in the second step, the molar ratio of the alcohol with respect to the crude fatty acid alkyl ester, calculated as an alcohol to fatty acid molar ratio is from 1.1 to 30, more preferably 1.5 to 25, and particularly preferably 2 to 20. In this manner, fatty acid alkyl esters can be efficiently manufactured from the fatty acid and/or triglyceride. If the molar ratio is less than 1.1, the esterification or ester exchange reaction will be insufficient, while if the molar ratio exceeds 40, the reaction equipment will be large, and the process energy consumption will also increase, which is not economical.

Note that, in the first step, the reaction may be performed with the addition of 0 to 3% of water with respect to the starting material oil or fat. This is suitable because, by causing water to be co-present with the reaction starting materials, the reaction rate is further increased. If the amount of water added with respect to the starting material oil or fat exceeds 3%, the reaction rate will in fact be lowered, which is undesirable.

### (ii) Second separation step (S20)

The first separation step is a step of removing the alcohol, glycerol and water from the fatty acid alkyl ester reaction solution A produced in the first reaction step, to produce a crude fatty acid alkyl ester A. The aforementioned fatty acid alkyl ester reaction solution A includes not only the fatty acid alkyl ester fraction principally comprising fatty acid alkyl esters, but also glycerol, water and alcohol that was added in excess.

In terms of methods for separating and removing the alcohol and water from the fatty acid alkyl ester solution A, simple distillation and rectification, making use of the boiling points of the fatty acid alkyl ester fraction, the alcohol and water may be mentioned. The simple distillation and rectification may be performed under ordinary pressure, or may be performed under reduced pressure. In the case of ordinary pressure distillation, this may be performed at a temperature higher than the boiling point of either the alcohol or the water. Furthermore, in the case of reduced pressure distillation, the distillation temperature may be suitably set in accordance with the degree of vacuum in the distillation apparatus. The higher the degree of vacuum in the distillation apparatus, the lower the distillation temperature at which it will be possible to perform distillation. Furthermore, in the present invention, the control target of the distillation operation is that the concentration of water contained in the crude fatty acid alkyl ester be no greater than 0.1%. This is suitable because, the lower the water content in the crude fatty acid alkyl ester A, the lower the acid value of the fatty acid alkyl ester produced in the second reaction step of reaction with alcohol.

The alcohol that is distilled out in the distillation step will be crude alcohol containing water, but if this crude alcohol is rectified using ordinary rectification methods, it is possible to produce ethanol with a purity of 99.8% or greater, which can be reused as a starting material for the present invention.

Note that, in terms of methods for separating and removing the glycerol from the fatty acid alkyl ester reaction solution A, precipitation separation, centrifugal separation, electrostatic separation and the like, which make use of the differences in specific weights and the differences in polarity between the fatty acid alkyl ester fraction and glycerol can be mentioned.

### (iii) Second reaction step (S30)

In the present invention, in the second reaction step, it is preferable that the alcohol and the crude fatty acid alkyl ester A, from which the water and the glycerol have been separated and removed, be contacted with the solid acid catalyst and reacted at a temperature of 60 to 210°C, at a pressure of 0.1 to 6.0 MPa. The animal or vegetable oils or fats used as starting materials normally contain free fatty acids. These free fatty acids react with alcohol to produce water as a secondary product. If water is present in the reaction system, the fee fatty acid alkyl ester produced may undergo hydrolysis and return to a free fatty acid. The higher the concentration of water in the reaction system, the more pronounced the hydrolysis reactions of the fatty acid alkyl ester. If the concentration of free fatty acids contained in the fatty acid alkyl ester exceeds a certain level, this may not conform to quality standards established for chemical products, biodiesel fuels and the like. If the free fatty acids remaining in the fatty acid alkyl ester contact the solid acid catalyst together with the alcohol, they can be converted to fatty acid alkyl ester. It is a matter of course that the monoglycerides, diglycerides and triglycerides remaining in the fatty acid alkyl ester can also be converted to fatty acid alkyl ester by way of an ester exchange reaction under the conditions of the second reaction step. By further converting the small amounts of free fatty acids and glycerides remaining in the fatty acid alkyl ester to fatty acid alkyl ester in this manner, the esterification rate can be improved and the yield and purity of the fatty acid alkyl ester that is the final product can be improved.

### (iv) Second separation step (S40)

Meanwhile, as well as the fatty acid alkyl ester, the crude fatty acid alkyl ester reaction solution B produced by the second reaction step contains the alcohol, glycerol and water. Here, in the second separation step, the alcohol, glycerol and water are separated and removed from the reaction solution, allowing a crude fatty acid alkyl ester B to be manufactured having a lower acid value and a higher purity.

The separation and removal of the alcohol, glycerol and water from the crude fatty acid alkyl ester reaction solution B can be performed by the same methods as in the first separation step. The alcohol from the separation step will be crude alcohol containing water, but if this crude alcohol is rectified using ordinary rectification methods, it is possible to produce ethanol with a purity of 99.8% or greater, which can be reused as a starting material for the present invention.

Furthermore, in the present invention, the control target for the glycerol separation operation is that the concentration of free glycerol contained in the crude fatty acid alkyl ester B be no greater than 0.02%. This is suitable because, the lower the free glycerol content in the crude fatty acid alkyl ester B, the higher the purity of the fatty acid alkyl ester that can be produced by subsequent refining steps.

### (v) Ester distillation step (S 50)

The ester distillation step is a step of distilling the crude fatty acid alkyl ester B produced in the second separation step under reduced pressure so as to cut the fractions having boiling points less than or equal to 100°C and greater than or equal to 360°C, to produce a refined fatty acid alkyl ester having a higher purity.

The crude fatty acid alkyl ester B may be used without modification for chemicals, biodiesel fuels and the like, but when higher purity is required, refinement by way of reduced pressure distillation is effective. In particular in the case of waste oil and fat starting materials, such as used tempura oil, the crude fatty acid alkyl ester produced in the second separation step may contain impurities such as the oxidative breakdown products of the oil or fat, thermal polycondensation products, and colored matter. These impurities are not converted to fatty acid alkyl esters by the method of manufacturing fatty acid alkyl esters of the present invention, and remain in the fatty acid alkyl ester phase. In the step of distilling the crude fatty acid alkyl ester under reduced pressure, the aforementioned impurities are cut as light components with boiling points less than or equal to 100°C and as heavy components having boiling points of greater than or equal to 360°C, so as to be removed from the crude fatty acid alkyl ester B.

Reduced pressure distillation of the crude fatty acid alkyl ester B is performed at no greater than 2000 Pa (15 Torr), and more preferably no greater than 660 Pa (5 Torr), and conditions such as the distillation temperatures are set so as to distill the fatty acid alkyl esters having boiling points of no less than 100°C and no greater than 360°C.

### Working Examples

Hereafter, the present invention will be described by way of working examples, but the present invention is in no way limited to these working examples. Examples 7, 8, 16 and 17 are not in accordance with the invention.

### Working Example 1 (manufacturing catalyst A)

With 100 parts by weight of Boehmite (Taimei Chemicals Co., Ltd.) were kneaded 5 parts by weight of 40% nitric acid and 100 parts by weight of distilled water, and after extrusion molding, this was fired for 6 hours at 500°C to produce a cylindrical molded γ-alumina carrier with a diameter of 1.2 mm (pore volume of 0.53 ml/g, specific surface area of 185 m²/g, average pore diameter of 75 Å).

In a glass beaker, 10.63 g of ammonium metatungstate ((NH₄)6H₂W₁₂O₄₀·nH₂O), 11.26 g of iron(III) sulfate (Fe₂(SO₄)₃) and 2.66 g of boric acid were dissolved in 110 g of distilled water, to prepare a mixed aqueous solution of ammonium metatungstate, iron(III) sulfate and boric acid.

100 g of the aforementioned γ-alumina carrier was impregnated with the mixed aqueous solution of ammonium metatungstate, iron(III) sulfate and boric acid in a glass beaker. This was subsequently dried at 120°C and fired for 6 hours at 500°C in a muffle furnace to prepare catalyst A.

### Working Example 2 (manufacturing catalyst B)

Other than using 15.24 g of cobalt(II) sulfate heptahydrate (CoSO₄·7H₂O) in place of the 11.26 g of iron(III) sulfate (Fe₂(SO₄)₃), catalyst B was prepared by way of the same method as in Working Example 1.

### Working Example 3 (manufacturing catalyst C)

Other than using 15.84 g of nickel(II) sulfate hexahydrate (NiSO₄·6H₂O) in place of the 11.26 g of iron(III) sulfate (Fe₂(SO₄)₃), catalyst C was prepared by way of the same method as in Working Example 1.

### Working Example 4 (manufacturing catalyst D)

Other than using 15.90 g of copper(II) sulfate heptahydrate (CuSO₄·7H₂O) in place of the 11.26 g of iron(III) sulfate (Fe₂(SO₄)₃), catalyst D was prepared by way of the same method as in Working Example 1.

### Working Example 5 (manufacturing catalyst E)

Other than using 8.92 g of zinc sulfate (Zn(SO₄)₂) in place of the 11.26 g of iron(III) sulfate (Fe₂(SO₄)₃), catalyst E was prepared by way of the same method as in Working Example 1.

### Working Example 6 (manufacturing catalyst F)

In a glass beaker, 12.26 g of hexaammonium molybdate tetrahydrate ((NH₄)6Mo₇O₂₄·4H2O), 8.92 g of zinc sulfate (ZnSO₄) and 2.66 g of boric acid (H₃BO₃) were dissolved in 110 g of water, to prepare a mixed aqueous solution of hexaammonium molybdate tetrahydrate, zinc sulfate and boric acid.

100 g of the aforementioned γ-alumina carrier was impregnated with the mixed aqueous solution of hexaammonium molybdate tetrahydrate, zinc sulfate and boric acid in a glass beaker by the same method as in Working Example 1. This was subsequently dried at 120°C and fired for 6 hours at 500°C in a muffle furnace to prepare catalyst F.

### Working Example 7 (manufacturing catalyst G)

In a glass beaker, 5.20 of ethyl silicate (Si(OC₂H₅)₄) was dissolved in 120 g of ethanol to prepare an ethanol solution of ethyl silicate.

100 g of the aforementioned γ-alumina carrier was impregnated with the ethanol solution of ethyl silicate in a glass beaker by the same method as in Working Example 1. Subsequently, this was dried at 120°C to produce a γ-alumina carrier supporting ethyl silicate.

In another beaker, 12.26 g of hexaammonium molybdate tetrahydrate ((NH₄)6Mo₇O₂₄·4H2O), 15.24 g of cobalt(II) sulfate heptahydrate (CoSO₄·7H₂O) were dissolved, to prepare a mixed aqueous solution of hexaammonium molybdate tetrahydrate and cobalt(II) sulfate heptahydrate.

The γ-alumina carrier supporting ethyl silicate was impregnated with the mixed aqueous solution of hexaammonium molybdate tetrahydrate and cobalt(II) sulfate heptahydrate in a glass beaker. This was subsequently dried at 120°C and fired for 6 hours at 500°C in a muffle furnace to prepare catalyst G.

### Working Example 8 (manufacturing catalyst H)

Other than using 7.78 g of tin(IV) chloride (SnCl₄) in place of the 15.24 g of cobalt(II) sulfate heptahydrate (CuSO₄·7H₂O), catalyst H was prepared by way of the same method as in Working Example 7.

### Working Example 9 (manufacturing catalyst I)

With 100 parts by weight of an mixture of Boehmite (Taimei Chemicals Co., Ltd.) and silicic acid n-hydrate (Wako Pure Chemical Industries, Ltd.) (Boehmite:silicic acid n-hydrate = 1:1), were kneaded 5 parts by weight of 40% nitric acid and 100 parts by weight of distilled water, and after extrusion molding, this was fired for 6 hours at 500°C, to produce a cylindrical molded silica-alumina carrier with a diameter of 1.2 mm (pore volume of 0.65 m²/g, specific surface area of 225 m²/g, average pore diameter of 105 Å).

In a glass beaker, 12.26 g of hexaammonium molybdate tetrahydrate ((NH₄)6Mo₇O₂₄·4H2O), 12.28 g of gallium(III) sulfate n-hydrate (Ga(SO₄)₃·nH₂O) and 2.66 g of boric acid (H₃BO₃) were dissolved in 110 g of water, to prepare a mixed aqueous solution of hexaammonium molybdate tetrahydrate, gallium(III) sulfate n-hydrate and boric acid.

The γ-alumina carrier supporting ethyl silicate was impregnated with the mixed aqueous solution of hexaammonium molybdate tetrahydrate, potassium(III) n-hydrate and boric acid in a glass beaker. This was subsequently dried at 120°C and fired for 6 hours at 500°C in a muffle furnace to prepare catalyst I.

### Comparative Example 1 (manufacture of comparative catalyst A)

10.63 g of ammonium metatungstate ((NH₄)6H₂W₁₂O₄₀·nH₂O) and 12.26 g of zinc nitrate hexahydrate (Zn(NO₃)₂·6H₂O) were dissolved in 110 g of water, to prepare a mixed aqueous solution of ammonium metatungstate and zinc nitrate hexahydrate.

A γ-alumina carrier prepared in the same manner as in Working Example 1 was impregnated with the mixed aqueous solution of ammonium metatungstate and zinc nitrate hexahydrate in a glass beaker. This was subsequently dried at 120°C and fired for 6 hours at 500°C in a muffle furnace to prepare comparative catalyst A.

### Comparative Example 2 (manufacture of comparative catalyst B)

10.63 g of ammonium metatungstate ((NH₄)6H₂W₁₂O₄₀·nH₂O) were dissolved in 110 g of distilled water, to prepare an aqueous solution of ammonium metatungstate.

A γ-alumina carrier prepared in the same manner as in Working Example 1 was impregnated with the aqueous solution of ammonium metatungstate, in a glass beaker. This was subsequently dried at 120°C and fired for 6 hours at 500°C in a muffle furnace to prepare comparative catalyst B.

### Comparative Example 3

Other than using 12.26 g of hexaammonium molybdate tetrahydrate ((NH₄)6Mo₇O₂₄·4H₂O) in place of the 10.63 g of ammonium metatungstate ((NH₄)6H₂W₁₂O₄₀·nH₂O), a comparative catalyst C was prepared in the same manner as in Comparative Example 2.

Working Examples 10 to 18 and Comparative Examples 4 to 6 (evaluation of catalyst performance)
50 g of a mixture of palm oil and palm fatty acid distillate (PFAD) (acid value of 120 mg KOH/g, water content of 0.06%), 4.16 g of methanol and 3.0 g of powders of the catalysts prepared in Working Examples 1 to 9 and Comparative Examples 1 to 3 were added to a TPR-2 type portable reactor reaction vessel made Taiatsu Techno Corporation, and this was covered and sealed. This was stirred for 1.5 hours at 150°C and a pressure of 0.8 MPa to produce a fatty acid methyl ester reaction solution. The reaction solution was left to stand, and separated into a methanol layer and an ester layer. Observations were made as to whether or not metal had been eluted from the catalyst by way of colorimetric analysis of the methanol layer, and the TG conversion ratio and FFA residue ratio were respectively found by GC analysis and acid value measurement of the ester layer. The results are shown in Table 1.

**[Table 1]**

| | catalyst | TG conversation rate (%) | FFA conversion rate (%) | metal elution |
|---|---|---|---|---|
| Working Example 10 | Catalyst A (10% WO₃ - 4.5% Fe₂O₃ -1.5%B₂O₃/Al₂O₃) | 90.3 | 95.2 | good |
| Working Example 11 | Catalyst B (10% WO₃ - 4.5% Co₃O₄ - 1.5%B₂O₃/Al₂O₃) | 86.8 | 91.5 | good |
| Working Example 12 | Catalyst C (10% WO₃ - 4.5% NiO - 1.5% B₂O₃/Al₂O₃) | 89.6 | 94.5 | good |
| Working Example 13 | Catalyst D (10% WO₃ - 4.5% CuO - 1.5%B₂O₃/Al₂O₃) | 88.7 | 93.5 | fair |
| Working Example 14 | Catalyst E (10% WO₃ - 4.5% ZnO - 1.5%B₂O₃/Al₂O₃) | 90.6 | 95.5 | very good |
| Working Example 15 | Catalyst F (10% MoO₃ - 4.5% ZnO - 1.5%B₂O₃/Al₂O₃) | 88.8 | 93.6 | good |
| Working Example 16 | Catalyst G (10% MoO₃ - 4.5% Co₃O₄ - 1.5%SiO₂/Al₂O₃) | 87.0 | 91.2 | very good |
| Working example 17 | Catalyst H (10% MoO₃ - 4.5% SnO₂ - 1.5%SiO₂/Al₂O₃) | 98.5 | 97.4 | very good |
| Working Example 18 | Catalyst I (10% WO₃ - 4.5% Ga₂O₃ - 1.5%B₂O₃/SiO₂-Al₂O₃) | 94.2 | 99.3 | good |
| Comparative Example 4 | Catalyst J (10% WO₃ - 4.5% ZnO/Al₂O₃) | 88.1 | 92.6 | poor |
| Comparative Example 5 | Catalyst K (10% WO₃/Al₂O₃) | 72.7 | 58.8 | poor |
| Comparative Example 6 | Catalyst K (10% MoO₃/Al₂O₃) | 79.2 | 62.4 | poor |

| | | | | |
|---|---|---|---|---|
| very good: absolutely none, good: almost none, fair: some, poor: pronounced elution TG: triglycerides, FFA free fatty acid | | | | |

### (Working Example 19) manufacture of fatty acid methyl ester from waste edible oil starting material

The oil and fat used was waste edible oil recovered from ordinary households (2% free fatty acid, 0.9% water).

The alcohol used was methanol (99.8% pure).

The solid acid catalyst used was the catalyst A, prepared in Working Example 1.

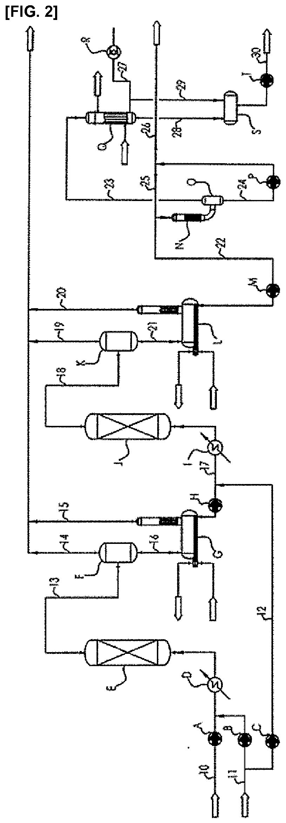

Manufacture of fatty acid methyl ester from waste edible oils was performed under the reaction conditions shown in Table 2, using the fixed bed type, high-pressure circulation reactor shown in FIG. 2. At 10 hours after starting the reaction, samples of crude fatty acid methyl ester A, crude fatty acid methyl ester B and refined fatty acid methyl ester were taken from the outlet of the No. 1 crude FAME transfer pump (H), the outlet of the No. 2 crude FAME transfer pump (M), and the outlet of the refined FAME transfer pump (T), and these were analyzed. The triglyceride (TG) conversion rate and the free fatty acid (FFA) residue rate found as a result of analyzing the crude fatty acid methyl ester A and the crude fatty acid methyl ester B are shown in Table 3, and the results of analyzing the properties of the refined fatty acid methyl ester are shown in Table 4.

**[Table 2]**

| Conditions | No. 1 reactor (E) | No. 2 reactor (J) |
|---|---|---|
| temperature | 210°C | 180°C |
| pressure | 4.5MPa | 4.5MPa |
| weight space velocity (WHSV) | 0.5h⁻¹ | 0.5h⁻¹ |
| alcohol/oil ratio | 0.54 (ratio by weight) | 0.54 (ratio by weight) |

**[Table 3]**

| Item | crude FAME-A | | crude FAME-B | |
|---|---|---|---|---|
| | TG conversion (%) | FFA (%) | TG conversion (%) | FFA (%) |
| Working Example 19 (waste edible oil) | 94.2 | 1.5 | 98.4 | 0.21 |
| Working Example 20 (crude palm) | 95. | 1.4 | 98.8 | 0.19 |
| Working Example 21 (PFAD) | 96.3 | 3.8 | 99.3 | 0.24 |

**[Table 4]**

| Item | | Working Example 19 (waste edible oil) | Working Example 20 (palm) | Working Example 21 (PFAD) |
|---|---|---|---|---|
| ester content | mass% | 98.1 | 99.4 | 98.4 |
| density (15°C) | g/cm³ | 0.8849 | 0.8758 | 0.8748 |
| kinematic viscosity | mm²/s | 4.178 | 4.501 | 4.409 |
| flash point | °C | 165.0 | 180.0 | 166.0 |
| sulfur content | mass% | 0.0006 | <0.0001 | 0.0003 |
| residual carbon content of 10% residual oil | mass% | 0.07 | 0.05 | 0.06 |
| cetane number | | 50.9 | 56.9 | - |
| sulfated ash | mass% | 0.001 | 0.001 | <0.001 |
| water content (Karl-Fischer method) | mg/kg | 320 | 370 | 100 |
| solid impurities | mg/kg | <1 | 0.5 | <1 |
| copper plate corrosion (50°C, 3 hr) | - | 1 | 1 | 1 |
| acid value stability (Rancimat method) | hr | 2.5 | 5.9 | 9.2 |
| acid value | mgKOH/g | 0.43 | 0.35 | 0.49 |
| sulfur value | - | 105 | 52 | 47 |
| iodine value | mass% | 5.0 | 0.3 | 0.4 |
| methyl linoleate | mass% | 0.37 | 0.00 | <0.01 |
| monoglyceride | mass% | 0.06 | 0.17 | 0.13 |
| diglyceride | mass% | <0.01 | 0.02 | <0.01 |
| triglyceride | mass% | <0.01 | 0.008 | <0.01 |
| free glycerol | mass% | 0.03 | 0.01 | 0.03 |
| total glycerol | mass% | 0.05 | 0.05 | 0.06 |
| metal (Na+K) | mg/kg | <1 | <1 | <1 |
| metal (Ca+Mg) | mg/kg | <1 | <1 | <1 |
| phosphorus | mg/kq | <3 | <3 | <3 |
| low temperature performance (fluid point) | °C | -2.5 | 12.5 | 15.0 |
| low temperature performance (clog point) | °C | -7 | 10 | 15 |

(Working Example 20) manufacture of fatty acid methyl ester from crude palm oil The oil used was crude palm oil (4.2% free fatty acid, 0.2% water).

The alcohol used was methanol (99.8% pure).

The solid acid catalyst used was the catalyst A, prepared in Working Example 1.

The manufacture of fatty acid methyl ester from crude palm oil was performed using the same apparatus as in Working Example 19, under the same reaction conditions as in Working Example 19. At 10 hours after starting the reaction, samples of crude fatty acid methyl ester A, crude fatty acid methyl ester B and refined fatty acid methyl ester were taken from the outlet of the No. 1 crude FAME transfer pump (H), the outlet of the No. 2 crude FAME transfer pump (M), and the outlet of the refined FAME transfer pump (T), and these were analyzed. The triglyceride (TG) conversion rate and the free fatty acid (FFA) residue rate found as a result of analyzing the crude fatty acid methyl ester A and the crude fatty acid methyl ester B are shown in Table 3, and the results of analyzing the properties of the refined fatty acid methyl ester are shown in Table 4.

### (Working Example 21) manufacture of fatty acid methyl ester from palm fatty acid distillate (PFAD)

The oil or fat used was palm fatty acid distillate (PFAD) (80% free fatty acid, 0.2% water). The alcohol used was methanol (99.8% pure). The solid acid catalyst used was the catalyst A, prepared in Working Example 1.

The manufacture of fatty acid methyl ester from PFAD was performed using the same apparatus as in Working Example 19, and under the same reaction conditions as in Working Example 19, other than the temperature of the No. 1 reactor and the No. 2 reactor being 200°C and 160°C respectively. At 10 hours after starting the reaction, samples of crude fatty acid methyl ester A, crude fatty acid methyl ester B and refined fatty acid methyl ester were taken from the outlet of the No. 1 crude FAME transfer pump (H), the outlet of the No. 2 crude FAME transfer pump (M), and the outlet of the refined FAME transfer pump (T), and these were analyzed. The triglyceride (TG) conversion rate and the free fatty acid (FFA) residue rate found as a result of analyzing the crude fatty acid methyl ester A and the crude fatty acid methyl ester B are shown in Table 3, and the results of analyzing the properties of the refined fatty acid methyl ester are shown in Table 4.

### Potential for Use in Industry

The solid acid catalyst of the present invention is useful in manufacturing high purity fatty acid alkyl esters that can be used as oleochemical starting materials or light oil alternative fuels, at low cost and high efficiency, from various oil and fat starting materials, including waste oils and fats discarded on large scales. Furthermore, this can be used as a catalyst for reactions requiring acid catalysts in the chemical industry such as alkylation reactions, acylation reactions, esterification reactions, isomerization reactions and the like. Conventionally, acid catalysts such as sulfuric acid, aluminium chloride, hydrogen fluoride, phosphoric acid, and p-toluenesulfonic acid have been used in these reactions, but the physical properties of these acid catalysts are such as to corrode metals and the use of expensive anticorrosion materials or anticorrosion processing was necessary. Furthermore, waste acid processing was necessary because separation from the reaction materials after the reaction was difficult, and complex processes such as alkali washing had to be undergone, which posed major problems from an environmental point of view. Furthermore, it was extremely difficult to reuse the catalyst. These problems are solved by using the solid acid catalyst of the present invention.

All of the publications, patents and patent applications cited in the present specification are directly incorporated by reference.

### Explanation of the Reference Numerals

- A: starting material oil supply pump
- B: first alcohol supply pump
- C: second alcohol supply pump
- D: No. 1 heater
- E: No. 1 reactor
- F: first gas liquid separator
- G: first methanol evaporation tank
- H: No. 1 crude FAME transfer pump
- I: No. 2 heater
- J: No. 2 reactor
- K: second gas-liquid separator
- L: second methanol evaporation tank
- M: No. 2 crude FAME transfer pump
- N: FAME heater
- O: FAME evaporation tank
- P: FAME circulation pump
- Q: condenser
- R: vacuum pump
- S: refined FAME tank
- T: refined FAME transfer pump
- 10 to 30: pipes

## Claims

1. A method of manufacturing a fatty acid alkyl ester by reacting a fatty acid and/or a triglyceride and an alcohol in the presence of a solid acid catalyst, the method of manufacturing a fatty acid alkyl ester being **characterized by** including:
a first reaction step of producing a fatty acid alkyl ester reaction solution A by contacting the fatty acid and/or the triglyceride and the alcohol with the solid acid catalyst at a temperature of 100 to 250°C and a pressure of 0.1 to 6.0 MPa;
a first separation step, following on said first reaction step, of removing said alcohol, water and glycerol from said fatty acid alkyl ester reaction solution A, to produce a crude fatty acid alkyl ester A having a water concentration of no greater than 0.1 mass%;
a second reaction step of contacting said crude fatty acid alkyl ester A and said alcohol with the solid acid catalyst at a temperature of 60 to 210°C at a pressure of 0.1 to 6.0 MPa; and
a second separation step of further removing said alcohol, water and glycerol from a fatty acid alkyl ester B produced in the second reaction step, to produce a fatty acid alkyl ester B having a free glycerol concentration of no greater than 0.02 mass%;
wherein the solid acid catalyst is produced by impregnating, on at least one inorganic porous carrier (A) selected from the group consisting of silica, alumina, or a combination thereof; an oxide (B) of at least one metallic element selected from the VIb group of the periodic table; an oxide or sulfate (C) of at least one metallic element selected from the group consisting of manganese (Mn), iron (Fe), cobalt (Co), nickel (Ni), copper (Cu), zinc (Zn), gallium (Ga) and tin (Sn); and an oxide (D) of at least one nonmetallic element selected from boron and silicon, **characterized in that** said oxide (B), oxide or sulfate (C) and oxide (D) are simultaneously impregnated to said inorganic porous carrier, wherein, with respect to the inorganic porous carrier (A), the amounts of the metal oxide (B), the metal oxide or sulfate (C) and the nonmetal oxide (D) impregnated, as calculated for the highest metal oxide, are 2.5 to 25 mass%, 1 to 10 mass%, and 0.5 to 5 mass%, respectively, and the total of (B), (C) and (D) is no greater than 30 mass%.

2. The method of manufacturing a fatty acid alkyl ester recited in claim 1, **characterized by** including an ester distillation step of distilling the crude fatty acid alkyl ester B produced in the second separation step under reduced pressure so as to cut the fractions having boiling points less than or equal to 100 °C and greater than or equal to 360 °C, to produce a refined fatty acid alkyl ester.

3. The method of manufacturing a fatty acid alkyl ester recited in claim 1 or claim 2, **characterized in that**, in said first reaction step, the molar ratio of the alcohol with respect to the fatty acid and/or triglyceride, calculated as an alcohol to fatty acid molar ratio, is from 1.2 to 40, and in said second step, the molar ratio of the alcohol with respect to the crude fatty acid alkyl ester, calculated as an alcohol to fatty acid molar ratio is from 1.1 to 30.

4. The method of manufacturing a fatty acid alkyl ester recited in any of claim 1 to claim 3, **characterized in that**, in said first separation step and said second separation step, the glycerol is separated after heating the fatty acid alkyl ester reaction solution A or the fatty acid alkyl ester reaction solution B to a temperature higher than the boiling point of either said alcohol or water at ordinary pressure or under reduced pressure, and evaporating said alcohol and the water.

5. The method of manufacturing a fatty acid alkyl ester recited in any of claim 1 to claim 4, **characterized in that**, in said first reaction step, the reaction is performed with the addition of 0 to 3 mass% water, with respect to the fatty acid and/or triglyceride that is the starting material.

## Patentansprüche

1. Verfahren zur Herstellung eines Fettsäurealkylesters durch Umsetzen einer Fettsäure und/oder eines Triglycerids und eines Alkohols in Gegenwart eines festen Säurekatalysators, wobei das Verfahren zur Herstellung eines Fettsäurealkylesters **dadurch gekennzeichnet ist, dass** es umfasst:
einen ersten Reaktionsschritt zum Ausbilden einer Fettsäurealkylester-Reaktionslösung A durch in Kontakt bringen der Fettsäure und/oder des Triglycerids und des Alkohols mit dem festen Säurekatalysator bei einer Temperatur von 100 bis 250 °C und einem Druck von 0,1 bis 6,0 MPa;
einen ersten Trennungsschritt, der dem ersten Reaktionsschritt folgt, zum Entfernen von Alkohol, Wasser und Glycerin aus der Fettsäurealkylester-Reaktionslösung A, so dass ein roher Fettsäurealkylester A mit einer Wasserkonzentration von nicht mehr als 0,1 Massen-% erhalten wird;
einen zweiten Reaktionsschritt zum in Kontakt bringen des rohen Fettsäurealkylesters A und des Alkohols mit dem festen Säurekatalysator bei einer Temperatur von 60 bis 210 °C und einem Druck von 0,1 bis 6,0 MPa; und
einen zweiten Trennungsschritt zum weiteren Entfernen von Alkohol, Wasser und Glycerin aus einem Fettsäurealkylester B, der im zweiten Reaktionsschritt gebildet wurde, so dass ein Fettsäurealkylester B mit einer freien Glycerinkonzentration von nicht mehr als 0,02 Massen-% erhalten wird;
wobei der feste Säurekatalysator gebildet wird durch Imprägnieren, auf mindestens einem anorganischen porösen Träger (A) ausgewählt aus der Gruppe bestehend aus Siliciumoxid, Aluminiumoxid oder einer Kombination davon, mit einem Oxid (B) von mindestens einem metallischen Element ausgewählt aus der Gruppe VIb des Periodensystems; einem Oxid oder Sulfat (C) von mindestens einem metallischen Element ausgewählt aus der Gruppe bestehend aus Mangan (Mn), Eisen (Fe), Cobalt (Co), Nickel (Ni), Kupfer (Cu), Zink (Zn), Gallium (Ga) und Zinn (Sn); und einem Oxid (D) von mindestens einem nichtmetallischen Element ausgewählt aus Bor und Silicium,
**dadurch gekennzeichnet, dass**
das Oxid (B), Oxid oder Sulfat (C) und Oxid (D) gleichzeitig in den anorganischen porösen Träger imprägniert werden,
wobei, bezogen auf den anorganischen porösen Träger (A), die imprägnierten Mengen an Metalloxid (B), Metalloxid oder-sulfat (C) und Nichtmetalloxid (D), berechnet für das höchste Metalloxid, 2,5 bis 25 Massen-%, 1 bis 10 Massen-% bzw. 0,5 bis 5 Massen% betragen, und der Gesamtbetrag an (B), (C) und (D) nicht mehr als 30 Massen-% beträgt.

2. Verfahren zur Herstellung eines Fettsäurealkylesters nach Anspruch 1, **dadurch gekennzeichnet, dass** es einen Esterdestillationsschritt zum Destillieren des rohen Fettsäurealkylesters B, der im zweiten Trennungsschritt erhalten wurde, unter reduziertem Druck umfasst, so dass die Fraktionen mit Siedepunkten von unter oder gleich 100 °C und über oder gleich 360 °C abgeschieden werden, so dass ein raffinierter Fettsäurealkylester erhalten wird.

3. Verfahren zur Herstellung eines Fettsäurealkylesters nach Anspruch 1 oder Anspruch 2, **dadurch gekennzeichnet, dass** im ersten Reaktionsschritt das Molverhältnis von Alkohol zu Fettsäure und/oder Triglycerid, berechnet als Molverhältnis von Alkohol zu Fettsäure, von 1,2 bis 40 beträgt, und im zweiten Schritt das Molverhältnis von Alkohol zu rohem Fettsäurealkylester, berechnet als Molverhältnis von Alkohol zu Fettsäure, von 1,1 bis 30 beträgt.

4. Verfahren zur Herstellung eines Fettsäurealkylesters nach einem der Ansprüche 1 bis 3, **dadurch gekennzeichnet, dass** im ersten Trennungsschritt und im zweiten Trennungsschritt das Glycerin nach Erhitzen der Fettsäurealkylester-Reaktionslösung A oder der Fettsäurealkylester-Reaktionslösung B auf eine Temperatur über dem Siedepunkt von Alkohol bzw. Wasser bei Normaldruck oder unter reduziertem Druck abgetrennt und der Alkohol und das Wasser verdampft wird.

5. Verfahren zur Herstellung eines Fettsäurealkylesters nach einem der Ansprüche 1 bis 4, **dadurch gekennzeichnet, dass** im ersten Reaktionsschritt die Umsetzung unter Zugabe von 0 bis 3 Massen-% Wasser, bezogen auf die Fettsäure und/oder das Triglycerid, die als Ausgangsmaterial verwendet werden, durchgeführt wird.

## Revendications

1. Procédé de fabrication d'un ester alkylique d'acide gras en faisant réagir un acide gras et/ou un triglycéride et un alcool en présence d'un catalyseur acide solide, le procédé de fabrication d'un ester alkylique d'acide gras étant **caractérisé en ce qu'**il comprend :
une première étape de réaction consistant à produire une solution de réaction A d'ester alkylique d'acide gras en mettant en contact l'acide gras et/ou le triglycéride et l'alcool avec le catalyseur acide solide à une température de 100 à 250°C et une pression de 0,1 à 6,0 MPa ;
une première étape de séparation, à la suite de ladite première étape de réaction, consistant à retirer lesdits alcool, eau et glycérol de ladite solution de réaction A d'ester alkylique d'acide gras, pour produire un ester alkylique d'acide gras brut A ayant une concentration en eau non supérieure à 0,1 % en masse ;
une seconde étape de réaction consistant à mettre en contact ledit ester alkylique d'acide gras brut A et ledit alcool avec le catalyseur acide solide à une température de 60 à 210°C à une pression de 0,1 à 6,0 MPa ; et
une seconde étape de séparation consistant à retirer davantage lesdits alcool, eau et glycérol d'un ester alkylique d'acide gras B produit lors de la seconde étape de réaction, pour produire un ester alkylique d'acide gras B ayant une concentration en glycérol libre non supérieure à 0,02 % en masse ;
dans lequel le catalyseur acide solide est produit en imprégnant, sur au moins un support poreux inorganique (A) choisi parmi le groupe constitué de silice, alumine, ou d'une combinaison de ceux-ci ; un oxyde (B) d'au moins un élément métallique choisi parmi le groupe VIb du tableau périodique ; un oxyde ou sulfate (C) d'au moins un élément métallique choisi parmi le groupe constitué de manganèse (Mn), fer (Fe), cobalt (Co), nickel (Ni), cuivre (Cu), zinc (Zn), gallium (Ga) et étain (Sn) ; et un oxyde (D) d'au moins un élément non métallique choisi parmi du bore et du silicium, **caractérisé en ce que** lesdits oxyde (B), oxyde ou sulfate (C) et oxyde (D) sont simultanément imprégnés sur ledit support poreux inorganique,
dans lequel, par rapport au support poreux inorganique (A), les quantités de l'oxyde métallique (B), de l'oxyde ou sulfate métallique (C) et de l'oxyde non métallique (D) imprégnés, telles que calculées pour l'oxyde métallique le plus élevé, sont de 2,5 à 25 % en masse, 1 à 10 % en masse, et 0,5 à 5 % en masse, respectivement, et le total de (B), (C) et (D) n'est pas supérieur à 30 % en masse.

2. Procédé de fabrication d'un ester alkylique d'acide gras selon la revendication 1, **caractérisé en ce qu'**il comprend une étape de distillation d'ester consistant à distiller l'ester alkylique d'acide gras brut B produit lors de la seconde étape de séparation sous pression réduite de manière à couper les fractions ayant des points d'ébullition inférieurs ou égaux à 100°C et supérieurs ou égaux à 360°C, pour produire un ester alkylique d'acide gras raffiné.

3. Procédé de fabrication d'un ester alkylique d'acide gras selon la revendication 1 ou la revendication 2, **caractérisé en ce que**, lors de ladite première étape de réaction, le rapport molaire de l'alcool par rapport à l'acide gras et/ou triglycéride, calculé sous forme d'un rapport molaire entre l'alcool et l'acide gras, est de 1,2 à 40, et lors de ladite seconde étape, le rapport molaire de l'alcool par rapport à l'ester alkylique d'acide gras brut, calculé sous forme d'un rapport molaire entre l'alcool et l'acide gras est de 1,1 à 30.

4. Procédé de fabrication d'un ester alkylique d'acide gras selon l'une quelconque des revendications 1 à 3, **caractérisé en ce que**, lors de ladite première étape de séparation et ladite seconde étape de séparation, le glycérol est séparé après chauffage de la solution de réaction A d'ester alkylique d'acide gras ou la solution de réaction B d'ester alkylique d'acide gras jusqu'à une température supérieure au point d'ébullition dudit alcool ou de l'eau à pression ordinaire ou sous pression réduite, et évaporation dudit alcool et de l'eau.

5. Procédé de fabrication d'un ester alkylique d'acide gras selon l'une quelconque des revendications 1 à 4, **caractérisé en ce que**, lors de ladite première étape de réaction, la réaction est effectuée avec l'ajout de 0 à 3 % en masse d'eau, par rapport à l'acide gras et/ou triglycéride qui est la matière de départ.
